(19) 

**Europäisches Patentamt**
**European Patent Office**
**Office européen des brevets**

(11) **EP 4 495 876 A1**

(12) **EUROPEAN PATENT APPLICATION**
published in accordance with Art. 153(4) EPC

(43) Date of publication:
**22.01.2025 Bulletin 2025/04**

(21) Application number: **22931333.3**

(22) Date of filing: **15.03.2022**

(51) International Patent Classification (IPC):
***G06T 7/00*** *(2017.01)*

(52) Cooperative Patent Classification (CPC):
**G06T 7/00**

(86) International application number:
**PCT/CN2022/080984**

(87) International publication number:
**WO 2023/173294 (21.09.2023 Gazette 2023/38)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
- **Bioland Laboratory**
  **Guangzhou, Guangdong 510320 (CN)**
- **Sun Yat-Sen Memorial Hospital , Sun Yat-Sen
  University**
  **Guangzhou, Guangdong 510120 (CN)**

(72) Inventors:
- **DING, Yue**
  **Guangzhou, Guangdong 510320 (CN)**
- **LIU, Jiang**
  **Guangzhou, Guangdong 510320 (CN)**

- **MA, Teng**
  **Guangzhou, Guangdong 510320 (CN)**
- **LUO, Wenqiang**
  **Guangzhou, Guangdong 510320 (CN)**
- **CHEN, Xiaoyi**
  **Guangzhou, Guangdong 510320 (CN)**
- **LEI, Baiying**
  **Guangzhou, Guangdong 510320 (CN)**
- **CHEN, Zhong**
  **Guangzhou, Guangdong 510320 (CN)**
- **CHEN, Zhiwei**
  **Guangzhou, Guangdong 510320 (CN)**
- **XIA, Chunmei**
  **Guangzhou, Guangdong 510320 (CN)**
- **GUO, Peidong**
  **Guangzhou, Guangdong 510320 (CN)**

(74) Representative: **Herzog IP Patentanwalts GmbH
Steinstraße 16-18
40212 Düsseldorf (DE)**

(54) **MODEL TRAINING METHOD AND APPARATUS, AND DEVICE, STORAGE MEDIUM AND PROGRAM PRODUCT**

(57) Disclosed in the embodiments of the present disclosure are a model training method and apparatus, and a device, a storage medium and a program product. The model training method comprises: determining an initial multi-channel residual neural network model; acquiring a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data; and training the initial multi-channel residual neural network model by taking the multi-channel bone radio-frequency data as an input and taking the fracture evaluation value label as an output, so as to obtain a multi-channel residual neural network model. By means of the technical solution, features of an ultrasonic radio-frequency signal can be comprehensively and accurately extracted, and a multi-channel residual neural network model having relatively high robustness is obtained by means of training, which model can be applied to the field of fracture risk prediction, such that an effective prevention measure is taken in a timely manner to reduce the probability of the occurrence of osteoporotic fracture, thereby reducing the pain of a patient.

EP 4 495 876 A1

determining an initial multi-channel residual neural network model  $S101$

obtaining a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data  $S102$

training the initial multi-channel residual neural network model by taking the multi-channel bone radio-frequency data as input and taking the fracture evaluation value label corresponding to the multi-channel bone radio-frequency data as output, so as to obtain a multi-channel residual neural network model  $S103$

FIG. 1

**Description**

FIELD OF THE INVENTION

The present disclosure relates to the technical field of health data processing, and in particular, to a model training method, and device, apparatus, storage medium and program product thereof.

BACKGROUND OF THE INVENTION

Osteoporosis is a metabolic bone disease syndrome characterized by decreased bone mass and destruction of bone microstructure, which can lead to increased bone fragility and susceptibility to osteoporotic fractures. Quantitative ultrasound (QUS) is a bone mineral density measurement technique. Its working principle is to detect bone quality by using the different propagation speeds and attenuations of ultrasound in different bone components. As a non-ionizing technology, QUS has the advantages of low cost, portability, rapidity, and no ionizing radiation, and therefore has good promotional properties. Based on the ultrasonic radio-frequency (RF) signal transmitted to and received from the bone by QUS equipment, parameters such as speed of sound(SOS), broadband ultrasonic attenuation(BUA), stiffness index(SI), quantitative ultrasonic index (QUI) , etc. can be calculated and output. The above parameters are only some of the characteristic values in the ultrasound radio-frequency signal. If the analysis is only based on the above parameters, a large amount of other information related to bone quality in the ultrasound radio-frequency signal will be lost. However, the ultrasound radio-frequency signal is relatively complex, and it is currently difficult to clarify the potential key variables in the ultrasound radio-frequency signal that may be related to the risk of osteoporotic fractures. Therefore, a model that can more comprehensively and accurately extract the characteristics of ultrasonic radio-frequency signals is urgently needed.

SUMMARY OF THE INVENTION

Embodiments of the present disclosure provide a model training method, device, apparatus, storage medium and program product thereof.
In a first aspect, an embodiment of the present disclosure provides a model training method.
Specifically, the model training method comprises:

determining an initial multi-channel residual neural network model;
obtaining a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data;
training the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

In an implementation of the present disclosure, the multi-channel residual neural network model comprises a multi-channel residual sub-network, a multi-channel global average pooling layer, a cascade layer and a decision neural sub-network connected sequentially, wherein:

the multi-channel residual sub-network is used to extract multi-channel bone features of the multi-channel bone radio-frequency data;
the multi-channel global average pooling layer is used to utilize global information to perform dimensionality reduction processing on the multi-channel bone features;
the cascade layer is used to perform cascade processing on the output of the multi-channel global average pooling layer to obtain cascade bone features;
the decision neural sub-network is used to make decisions based on the cascade bone features.

In an implementation of the present disclosure, the multi-channel residual sub-network is composed of residual modules of a plurality of channels; the residual module of each channel comprises a plurality of cascaded residual blocks; each residual block comprises a convolution connection branch, a shortcut connection branch, an addition layer, an activation function layer and a max pooling layer; the convolution connection branch comprises a plurality of groups of convolution layers, activation function layers and batch normalization layers; the shortcut connection branch comprises a group of convolution layer, activation function layer and batch normalization layer; the convolution connection branch and the shortcut connection branch are aggregated in the addition layer, and an activation function layer and the max pooling layer are connected behind the addition layer;

the decision neural sub-network comprises two fully connected layers and an activation function layer, wherein the number of neurons in the first fully connected layer is within the preset number of neurons, and the number of neurons in the second fully connected layer is related to the number of clinical information data categories.

In an implementation of the present disclosure, said training the initial multi-channel residual neural network model by taking the multi-channel bone radio-frequency data as input and taking the fracture evaluation value label corresponding to the multi-channel bone radio-frequency data as output, comprises:

inputting the multi-channel bone radio-frequency data into a plurality of cascaded residual blocks of the residual module of the corresponding channel in the multi-channel residual sub-network, wherein the output of the end residual block is the multi-channel bone features corresponding to the multi-channel bone radio-frequency data;

inputting the multi-channel bone features into the multi-channel global average pooling layer;

inputting the output of the multi-channel global average pooling layer into the cascade layer to obtain the cascade bone features;

inputting the cascade bone features into the first fully connected layer and the second fully connected layer in the decision neural sub-network sequentially, and inputting the output of the second fully connected layer into the activation function layer to perform nonlinear calculation, so as to obtain a bone data decision value.

In an implementation of the present disclosure, said inputting the multi-channel bone radio-frequency data into a plurality of cascaded residual blocks of the residual module of the corresponding channel in the multi-channel residual sub-network comprises:

inputting the multi-channel bone radio-frequency data into the convolution connection branch and the shortcut connection branch of the first residual block of the residual module of the corresponding channel in the multi-channel residual sub-network;

inputting the output of the convolution connection branch and the output of the shortcut connection branch into the addition layer, wherein the outputs are added in the addition layer;

inputting the output of the addition layer into the activation function layer for processing;

inputting the output of the activation function layer into the max pooling layer, and inputting the output of the max pooling layer, which is used as the input of the next residual block, into the convolution connection branch and the shortcut connection branch of the next residual block, until reaching the end residual block.

In an implementation of the present disclosure, before inputting the multi-channel bone radio-frequency data into a plurality of cascaded residual blocks of the residual module of the corresponding channel in the multi-channel residual sub-network, the method further comprises:

preprocessing the multi-channel bone radio-frequency data.

In an implementation of the present disclosure, the multi-channel residual neural network model performs model training by taking small sample cross entropy as the loss function.

In an implementation of the present disclosure, the method further comprises:

obtaining multi-channel bone radio-frequency data to be decided;

inputting the multi-channel bone radio-frequency data to be decided into the multi-channel residual neural network model, to obtain the bone data decision value corresponding to the multi-channel bone radio-frequency data to be decided.

In an implementation of the present disclosure, the method further comprises:

performing a preset operation according to the bone data decision value.

In a second aspect, an embodiment of the present disclosure provides a model training device.

Specifically, the model training device comprises:

a determining module configured to determine an initial multi-channel residual neural network model;

a first obtaining module configured to obtain a multi-channel residual neural network model training dataset, wherein the multi-channel residual neural network model training data set comprises multi-channel bone radio-frequency data obtained by a QUS device, and a fracture evaluation value label corresponding to the multi-channel bone radio-frequency data;

a training module configured to train the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

In a third aspect, an embodiment of the present disclosure provides a fracture risk prediction device.

Specifically, the fracture risk prediction device comprises:

a second obtaining module configured to obtain multi-channel bone radio-frequency data obtained by a QUS device;
an input module configured to input the multi-channel bone radio-frequency data into a pre-trained multi-channel residual neural network model to obtain a fracture risk prediction probability;
a prediction module configured to obtain a fracture risk prediction result based on the fracture risk prediction probability.

In a fourth aspect, an embodiment of the present disclosure provides an electronic apparatus, comprising a memory and at least one processor, wherein the memory is used to store one or more computer instructions, wherein the one or more computer instructions are executed by the at least one processor to implement the steps of the model training method mentioned above.

In a fifth aspect, an embodiment of the present disclosure provides a computer-readable storage medium for storing computer instructions used by a model training device, which comprise computer instructions related to the model training device for executing the above model training method.

In a sixth aspect, an embodiment of the present disclosure provides a computer program product, comprising computer programs/instructions, wherein the computer programs/instructions implement the steps of the above-mentioned model training method when the computer programs/instructions are executed by a processor.

The technical solutions provided by the embodiments of the present disclosure may comprise the following beneficial effects:

The model training method proposed by the above technical solution can comprehensively and accurately extract the characteristics of ultrasonic radio-frequency signals, and train a robust multi-channel residual neural network model. This model can be applied in the field of fracture risk prediction to take timely and effective preventive measures, which can reduce the incidence of osteoporotic fractures and reduce patients' pain.

It should be understood that the above general description and the following detailed description are only exemplary and explanatory, and cannot limit the present disclosure.

DESCRIPTION OF THE DRAWINGS

With reference to the accompanying drawings, through the following detailed description of the nonlimiting embodiments, other features, purposes, and advantages of the present disclosure will become more apparent. In the accompanying drawings:

FIG. 1 shows a flow chart of a model training method according to an embodiment of the present disclosure;
FIG. 2A shows a structural diagram of a multi-channel residual sub-network according to an embodiment of the present disclosure;
FIG. 2B shows a structural block diagram of a residual block according to an embodiment of the present disclosure;
FIG. 2C shows an overall flow chart according to an embodiment of the present disclosure;
FIG. 3 shows a structural block diagram of a model training device according to an embodiment of the present disclosure;
FIG. 4 shows a structural block diagram of a fracture risk prediction device according to an embodiment of the present disclosure;
FIG. 5 shows a structural block diagram of an electronic apparatus according to an embodiment of the present disclosure;
FIG. 6 is a schematic structural diagram of a computer system suitable for implementing a model training method according to an embodiment of the present disclosure.

DETAILED DESCRIPTION

Hereinafter, exemplary embodiments of the present disclosure will be described in detail with reference to the accompanying drawings, so that those skilled in the art can easily implement them. In addition, for the sake of clarity, parts irrelevant to describing the exemplary embodiments are omitted in the drawings.

In the present disclosure, it should be understood that terms such as "comprise" or "have" are intended to indicate the existence of the features, numbers, steps, actions, components, parts or combinations thereof disclosed in this specification, and are not intended to exclude the possibility of existence or addition of one or more other features, numbers, steps, actions, components, parts or combinations thereof.

In addition, it should be noted that the embodiments in the present disclosure and the features in the embodiments can be combined with each other if there is no conflict. Hereinafter, the present disclosure will be described in detail with reference to the drawings and in conjunction with the embodiments.

The technical solution provided by the embodiments of the present disclosure can comprehensively and accurately extract the characteristics of ultrasonic radio-frequency signals, and train a robust multi-channel residual neural network model. This model can be applied in the field of fracture risk prediction to take timely and effective preventive measures, which can reduce the incidence of osteoporotic fractures and reduce patients' pain.

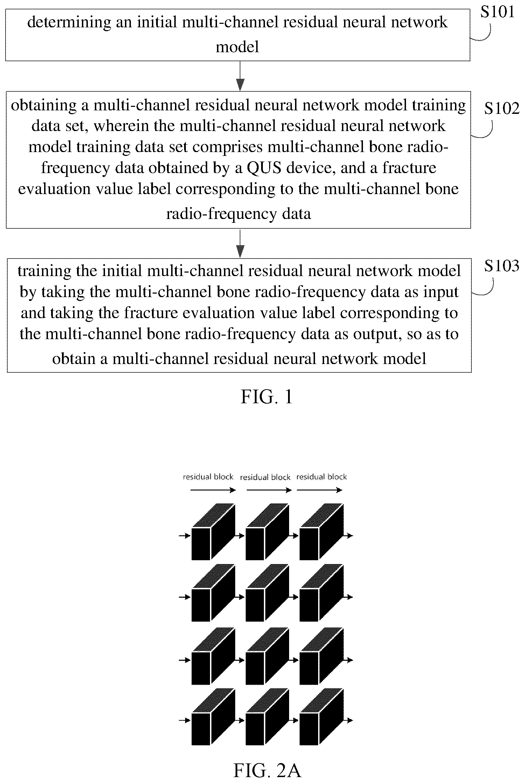

FIG. 1 shows a flow chart of a model training method according to an embodiment of the present disclosure. As shown in FIG. 1, the model training method comprises the following steps S101-S103:

In step S101, determining an initial multi-channel residual neural network model;

In step S102, obtaining a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data;

In step S103, training the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

As mentioned above, osteoporosis is a metabolic bone disease syndrome characterized by decreased bone mass and destruction of bone microstructure, which can lead to increased bone fragility and susceptibility to osteoporotic fractures. Quantitative ultrasound (QUS) is a bone mineral density measurement technique. Its working principle is to detect bone quality by using the different propagation speeds and attenuations of ultrasound in different bone components. As a non-ionizing technology, QUS has the advantages of low cost, portability, rapidity, and no ionizing radiation, and therefore has good promotional properties. Based on the ultrasonic radio-frequency (RF) signal transmitted to and received from the bone by QUS equipment, parameters such as sound velocity value, broadband ultrasonic attenuation value, rigidity index, quantitative ultrasonic index, etc. can be calculated and output. The above parameters are only some of the characteristic values in the ultrasound radio-frequency signal. If the analysis is only based on the above parameters, a large amount of other information related to bone quality in the ultrasound radio-frequency signal will be lost. However, the ultrasound radio-frequency signal is relatively complex, and it is currently difficult to clarify the potential key variables in the ultrasound radio-frequency signal that may be related to the risk of osteoporotic fractures. Therefore, a model that can more comprehensively and accurately extract the characteristics of ultrasonic radio-frequency signals is urgently needed.

Taking into account the above defects, in this embodiment, a model training method is proposed, which can comprehensively and accurately extract the characteristics of ultrasonic radio-frequency signals, and train a robust multi-channel residual neural network model. This model can be applied in the field of fracture risk prediction to take timely and effective preventive measures, which can reduce the incidence of osteoporotic fractures and reduce patients' pain.

In an embodiment of the present disclosure, the model training method can be applied to model training parties such as computers, computing devices, terminal devices, electronic apparatus, servers, and service clusters that train models.

In an embodiment of the present disclosure, the initial multi-channel residual neural network model refers to a multi-channel residual neural network model, which has initial parameters and is used as a prototype, for model training.

In an embodiment of the present disclosure, the multi-channel residual neural network model training data set refers to a set composed of data used to train the multi-channel residual neural network model. Herein the multi-channel residual neural network model training data set comprises multi-channel bone radio-frequency data obtained by a QUS device which can be used as the model training input, and a fracture evaluation value label corresponding to the multi-channel bone radio-frequency data which can be used as the model training output.

In the embodiments, the multi-channel bone radio-frequency data obtained by a QUS device refers to the ultrasonic radio-frequency signal data transmitted to and received from a bone by the QUS device, which usually comprises bone radio-frequency data collected by means of a plurality of channels. The number of the channels can be set according to the requirement of the actual application. For example, it can be set to 4. In an embodiment of the present disclosure, the multi-channel bone radio-frequency data can be multi-channel radius radio-frequency data.

In the embodiments, the fracture evaluation value label corresponding to the multi-channel bone radio-frequency data refers to the fracture evaluation value obtained based on the multi-channel bone radio-frequency data. For example, if the bone with the multi-channel bone radio-frequency data is in fracture status, then the fracture evaluation value label corresponding to the multi-channel bone radio-frequency data is 1. If the bone with the multi-channel bone radio-frequency data is not in a fracture status, then the fracture evaluation value label corresponding to the multi-channel bone radio-frequency data is 0.

In the above embodiments, when the multi-channel residual neural network model is trained, the initial multi-channel residual neural network model is first determined; then the multi-channel bone radio-frequency data obtained by a QUS device, and the fracture evaluation value label corresponding to the multi-channel bone radio-frequency data are obtained; hereafter, the initial multi-channel residual neural network model is trained, by taking the multi-channel bone radio-frequency data as input of the initial multi-channel residual neural network model, and by taking the fracture evaluation value label corresponding to the multi-channel bone radio-frequency data as output of the initial multi-channel residual neural network model. When the training results converge, the multi-channel residual neural network model is obtained.

In an embodiment of the present disclosure, the multi-channel residual neural network model performs model training by taking small sample cross entropy as the loss function. Herein the small sample cross entropy refers to a cross entropy function that can focus on a small number of sample categories and can solve the problem of data imbalance. The small sample cross entropy can be expressed as:

$$L = -w(1 - \tilde{p}) \log(p) \qquad (1)$$

$$w = \frac{B + S}{B} \qquad (2)$$

wherein $L$ represents the loss function of the multi-channel residual neural network model, $B$ represents the number of data categories with a larger number in the multi-channel residual neural network model training data set, $S$ represents the number of data categories with a less number in the multi-channel residual neural network model training data, $\tilde{p}$ represents the true category value of the training input data, and $p$ is the predicted probability value that the training input data is determined to be a fracture, that is, the fracture risk value.

When the initial multi-channel residual neural network model is trained by using the loss function, the stochastic gradient descent (SGD) method is used to minimize the loss function to optimize the model parameters of the initial multi-channel residual neural network model, so as to make the initial multi-channel residual neural network model have the best performance.

In an embodiment of the present disclosure, the multi-channel residual neural network model comprises a multi-channel residual sub-network, a multi-channel global average pooling layer, a cascade layer and a decision neural sub-network connected sequentially, wherein the multi-channel residual sub-network is used to extract multi-channel bone features of the multi-channel bone radio-frequency data; the multi-channel global average pooling layer is used to utilize global information to perform dimensionality reduction processing on the multi-channel bone features; the cascade layer is used to perform cascade processing on the output of the multi-channel global average pooling layer to obtain cascade bone features; the decision neural sub-network is used to make decisions based on the cascade bone features to obtain the fracture evaluation value corresponding to the multi-channel bone radio-frequency data.

In an embodiment of the present disclosure, the multi-channel residual sub-network refers to a residual network with a plurality of channels, which is designed with respect to the multi-channel bone radio-frequency data and is used to extract features of the bone radio-frequency data. Herein the number of channels of the multi-channel residual sub-network corresponds to the number of channels of the multi-channel bone radio-frequency data. For example, if the number of channels of the multi-channel bone radio-frequency data is 4, then the number of channels of the multi-channel residual sub-network is also 4, so that feature extraction can be performed on the bone radio-frequency data of each channel of the multi-channel bone radio-frequency data respectively.

Herein the multi-channel residual sub-network is composed of residual modules of a plurality of channels, and the residual module of each channel comprises a plurality of cascaded residual blocks. FIG. 2A shows a structural block diagram of a multi-channel residual sub-network according to an embodiment of the present disclosure. As shown in FIG. 2A, the multi-channel residual sub-network is composed of residual modules of 4 channels, and the residual module of each channel comprises 3 cascaded residual blocks.

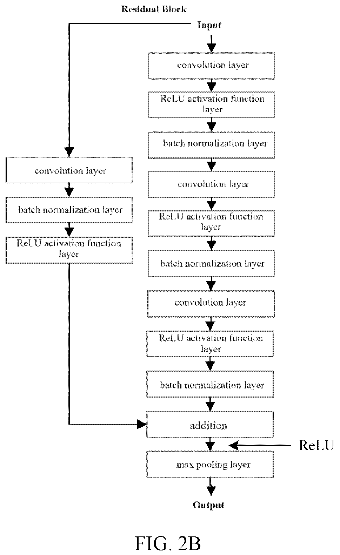

Herein each residual block comprises a convolution connection branch, a shortcut connection branch, an addition layer, an activation function layer and a max pooling layer; the convolution connection branch comprises a plurality of groups of convolution layers, activation function layers and batch normalization layer; the shortcut connection branch comprises a group of convolution layer, activation function layer and batch normalization layer; the convolution connection branch and the shortcut connection branch are aggregated in the addition layer, and an activation function layer and the max pooling layer are connected behind the addition layer. FIG. 2B shows a structural block diagram of a residual block according to an embodiment of the present disclosure. As shown in FIG. 2B, the residual block comprises a convolution connection branch, a shortcut connection branch, an addition layer, an activation function layer and a max pooling layer. Herein the convolution connection branch comprises 3 groups of sequentially connected 1-dimensional convolution layer, Rectified Linear Unit (ReLU) activation function layer and batch normalization (BN) layer. Furthermore, each convolution layer has 16 convolution kernels, and the lengths of the convolution kernels are 8, 5, and 3 respectively. The shortcut connection branch comprises one group of convolution layer, ReLU activation function layer and batch normalization layer. Furthermore, the convolution layer has 16 convolution kernels with a length of 1. The convolution connection branch and the shortcut connection branch are aggregated in the addition layer. That is, the output of the last batch normalization layer of the convolution connection branch and the output of the batch normalization layer of the shortcut connection branch are performed addition of the corresponding elements of the feature vectors in the addition layer. Then, after the output of the addition layer is input to the ReLU activation function layer for processing, the processing result is input to the max pooling layer, and finally the output of the residual block is obtained.

Herein the convolution operation of the convolution layer can implement feature extraction and obtain feature vectors. The activation function of the activation function layer can determine whether the output of each neuron reaches the threshold, that is, whether the feature strength of a certain part of the data reaches a certain standard. If the output of each neuron does not reach the threshold, it is set to 0, which indicates that the features extracted from this part of the data do not affect classification obviously, and these features can be determined not to be output. In addition, the ReLU activation function has a certain sparsity. The network model through sliming process by the ReLU activation function can mine relevant features effectively, fit the training data, and improve the expressive ability of the network model. The data batch normalization operation of the batch normalization layer can speed up the convergence speed of the network model training, make the training process of the network model more stable, avoid gradient explosion or gradient disappearance, and can play a certain role in regularization. On the one hand, the max pooling layer can compress the input feature vectors, extract the main features, and achieve under-sampling. On the other hand, it can also reduce the size of the network model, reduce the computational complexity of the network model, and the occurrence overfitting is prevented to a certain extent.

In an embodiment of the present disclosure, the decision neural sub-network refers to a model implemented based on neural network for making decision on fracture evaluation value.

Herein the decision neural sub-network comprises two fully connected layers and an activation function layer, wherein the activation function used in the activation function layer could be a sigmoid activation function. Herein the number of neurons in the first fully connected layer is within the preset neuron number range. The preset neuron number range can be set according to the needs of actual applications, for example, it can be set to 32-64. The number of neurons in the second fully connected layer is related to the number of clinical information data categories, that is, the number of neurons in the second fully connected layer can be adjusted according to whether the clinical information data is introduced. Herein the clinical information data corresponds to the multi-channel bone radio-frequency data. For example, the clinical information data and the multi-channel bone radio-frequency data belong to the same individual. For example, if the number of neurons in the second fully connected layer is preset to 10 and there is currently no need to add the clinical information data, then the number of neurons in the second fully connected layer will still be 10. If the number of neurons in the second fully connected layer is preset to 10, and currently three types of clinical information data need to be added: weight, height, and age, then the number of neurons in the second fully connected layer can be increased from 10 to 13. Furthermore, the activation function of the first fully connected layer can be a ReLU activation function, and the random deactivation ratio is 0.5.

In an embodiment of the present disclosure, step S 103, that is, the step of training the initial multi-channel residual neural network model by taking the multi-channel bone radio-frequency data as input and taking the fracture evaluation value label corresponding to the multi-channel bone radio-frequency data as output, could comprise the following steps:

inputting the multi-channel bone radio-frequency data into a plurality of cascaded residual blocks of the residual module of the corresponding channel in the multi-channel residual sub-network, wherein the output of the end residual block is the multi-channel bone features corresponding to the multi-channel bone radio-frequency data;
inputting the multi-channel bone features into the multi-channel global average pooling layer;
inputting the output of the multi-channel global average pooling layer into the cascade layer to obtain the cascade bone features;
inputting the cascade bone features into the first fully connected layer and the second fully connected layer in the decision neural sub-network sequentially, and inputting the output of the second fully connected layer into the activation function layer to perform nonlinear calculation, so as to obtain a bone data decision value.

In the embodiment, when the initial multi-channel residual neural network model is trained:
First, the multi-channel bone radio-frequency data is input into a plurality of cascaded residual blocks of the residual module of the corresponding channel in the multi-channel residual sub-network, and the multi-channel bone features corresponding to the multi-channel bone radio-frequency data is extracted. As mentioned above, the multi-channel residual sub-network is composed of residual modules of a plurality of channels; and the residual module of each channel comprises a plurality of cascaded residual blocks. Therefore, in the embodiment, when the multi-channel residual sub-network is used to extract multi-channel bone features corresponding to the multi-channel bone radio-frequency data, the multi-channel bone radio-frequency data can be input into a plurality of cascaded residual blocks of the residual module of the corresponding channel in the multi-channel residual sub-network respectively. The output of the final residual block of each channel are the multi-channel bone features corresponding to the multi-channel bone radio-frequency data, which is extracted from the multi-channel bone radio-frequency data.

Then, the multi-channel bone features extracted by means of the multi-channel residual sub-network are input into the multi-channel global average pooling layer, so as to utilize global information to perform dimensionality reduction processing on the multi-channel bone features respectively.

Then, the multi-channel bone features processed by the multi-channel global average pooling layer are input into the cascade layer, so that the multi-channel bone features are axially cascaded along the channel axis, and the features obtained by the cascade will subsequently be used as the input of the multi-channel residual neural network model.

Finally, the cascade bone features are input into the first fully connected layer and the second fully connected layer in the decision neural sub-network sequentially, and the output of the second fully connected layer is input into the activation function layer to perform nonlinear calculation, so as to obtain a bone data decision value.

Specifically, in the embodiment, assuming that the number of channels K is 4, when the output of the cascade layer is input into the decision neural sub-network, the processing of the first fully connected layer can be expressed as :

$$f_n = w_n z + b_n \qquad\qquad (3)$$

$$z = C_k^{K=4} z_k \qquad\qquad (4)$$

wherein $f_n$ represents the output of the n-th neuron in the first fully connected layer, $w_n$ and $b_n$ represents the weight parameter and the bias value of the first fully connected layer respectively, $z_k$ represents the output of the k-th channel residual module through the global average pooling layer, $z$ represents the output obtained after cascading the outputs $z_k$ of all channels.

Inputting the output of the first fully connected layer to the second fully connected layer to perform processing is similar to the processing of the first fully connected layer. It should be noted that the input of the second fully connected layer comprises the output of the first fully connected layer, in addition may also comprise clinical information data. When the input of the second fully connected layer comprises the clinical information data, the output of the first fully connected layer and the clinical information data can be jointly input to the second fully connected layer for processing.

Wherein the bone data decision value refers to a value that can be used to make decisions on the bone data, for example, a fracture evaluation value expressed as a probability value.

In an embodiment of the present disclosure, the step of inputting the multi-channel bone radio-frequency data into a plurality of cascaded residual blocks of the residual module of the corresponding channel in the multi-channel residual sub-network may comprise the following step:

inputting the multi-channel bone radio-frequency data into the convolution connection branch and the shortcut connection branch of the first residual block of the residual module of the corresponding channel in the multi-channel residual sub-network;

inputting the output of the convolution connection branch and the output of the shortcut connection branch into the addition layer, wherein the outputs are added in the addition layer;

inputting the output of the addition layer into the activation function layer for processing;

inputting the output of the activation function layer into the max pooling layer, and inputting the output of the max pooling layer, which is used as the input of the next residual block, into the convolution connection branch and the shortcut connection branch of the next residual block, until reaching the end residual block.

As mentioned above, each residual block comprises a convolution connection branch, a shortcut connection branch, an addition layer, an activation function layer and a max pooling layer; the convolution connection branch comprises a plurality of groups of convolution layers, activation function layers and batch normalization layer; the shortcut connection branch comprises a group of convolution layer, activation function layer and batch normalization layer; the convolution connection branch and the shortcut connection branch are aggregated in the addition layer, and an activation function layer and the max pooling layer are connected behind the addition layer. Therefore, in the embodiment, when the multi-channel bone radio-frequency data is input into a plurality of cascaded residual blocks of the residual module of the corresponding channel in the multi-channel residual sub-network, first, the multi-channel bone radio-frequency data is input into the convolution connection branch and the shortcut connection branch of the first residual block of the residual module of the corresponding channel in the multi-channel residual sub-network, to perform convolution processing and mapping processing respectively. Then, the output of the convolution connection branch and the output of the shortcut connection branch are input into the addition layer, the addition of the corresponding elements of the feature vectors are performed in the addition layer. Then, the output of the addition layer is input into the activation function layer for processing. After that, the output of the activation function layer is input into the max pooling layer, and the output of the max pooling layer can be used as the input of the next residual block, which is input to the convolution connection branch and shortcut connection branch of the next residual block. Subsequent processing can be performed in a similar manner, until reaching the end residual block, the output of the end residual block is the multi-channel bone features corresponding to the multi-channel bone radio-frequency data.

Assume that the multi-channel bone radio-frequency data is expressed as: $R_t = (R_{1t}, ..., R_{kt} ..., R_{Kt})^T$, wherein $T$ represents transposition, K represents the number of channels, $R_{kt}$ represents the bone radio-frequency time series of the k-th channel, as shown in the following:

$$R_{kt} = \{r_{k1}, r_{k2}, ..., r_{kt}, ..., r_{kL}\} \qquad (5)$$

wherein $r_{kt}$ represents the data value of the k-th channel at the t-th time point, and $L$ represents the total length of the time series.

For the bone radio-frequency time series $R_{kt}$ of the k-th channel, the output after processing of the residual block can be expressed as:

$$v_{l+1,k} = v_{l,k} * W_{l,k}^r + \mathcal{F}(v_{l,k}, W_{l,k}^c) \qquad (6)$$

$$y_{l+1,k} = f(v_{l+1,k}) \qquad (7)$$

wherein $v_{l,k}$ represents the input of the *l*-th residual block; $W_{l,k}^r$ and $W_{l,k}^c$ represent the weights of the shortcut connection branch and the convolution connection branch in the *l*-th residual block respectively; ( ... ) represents the skip connection processing; $f$( ... ) represents the ReLU activation function and the max pooling processing; $y_{l+1,k}$ represents the output obtained after $v_{l,k}$ is processed by means of the ReLU activation function and the max pooling in the *l*-th residual block, that is, the output of the *l*-th residual block, which can also be considered as the input of the *l*+1-th residual block. When *l* =0, $v_{l,k}$ is the bone radio-frequency time series $R_{kt}$ of the *k*-th channel.

In an embodiment of the present disclosure, before inputting the multi-channel bone radio-frequency data into a plurality of cascaded residual blocks of the residual module of the corresponding channel in the multi-channel residual sub-network, the method may further comprise:

preprocessing the multi-channel bone radio-frequency data.

In order to facilitate feature extraction of the multi-channel bone radio-frequency data, in this embodiment, before inputting the multi-channel bone radio-frequency data into a plurality of cascaded residual blocks of the residual module of the corresponding channel in the multi-channel residual sub-network, the multi-channel bone radio-frequency data is preprocessed. Herein the preprocessing may comprise one or more of the following: screening of valid data, data sampling, and data normalization. etc. For example, assuming that the raw bone radio-frequency data of each channel has 1024 time points, according to the validity of the data, the data of 725 valid time points can be selected. Then the data of 625 time points can be obtained through data sampling. Finally, the obtained data is converted into a value with an amplitude in the range of 0-255.

In an embodiment of the present disclosure, the method may further comprise the following steps:

obtaining multi-channel bone radio-frequency data to be decided;
inputting the multi-channel bone radio-frequency data to be decided into the multi-channel residual neural network model, to obtain the bone data decision value corresponding to the multi-channel bone radio-frequency data to be decided.

In this embodiment, when the multi-channel residual neural network model is used to make bone data decision, first the multi-channel bone radio-frequency data to be decided is obtained. Then the multi-channel bone radio-frequency data to be decided is input into the multi-channel residual neural network model, to perform feature extraction and bone data decision-making. Finally the bone data decision value corresponding to the multi-channel bone radio-frequency data to be decided is obtained.

In an embodiment of the present disclosure, the method may further comprise the following steps:
performing a preset operation according to the bone data decision value.

As mentioned above, the bone data decision value can be expressed as a probability value. Therefore, in this embodiment, the bone data decision value can be used to perform a preset operation such as fracture risk evaluation. For example, the bone data decision value can be compared with a preset probability threshold to obtain the bone data decision result. Herein the preset probability threshold can be set according to the need of actual application before the multi-channel residual neural network model training. For example, the preset probability threshold can be set to 50%. At this time, if the bone data decision value is higher than 50%, it can be evaluated that there is a risk of fracture. If the bone data decision value is lower than 50%, it can be evaluated that there is no risk of fracture.

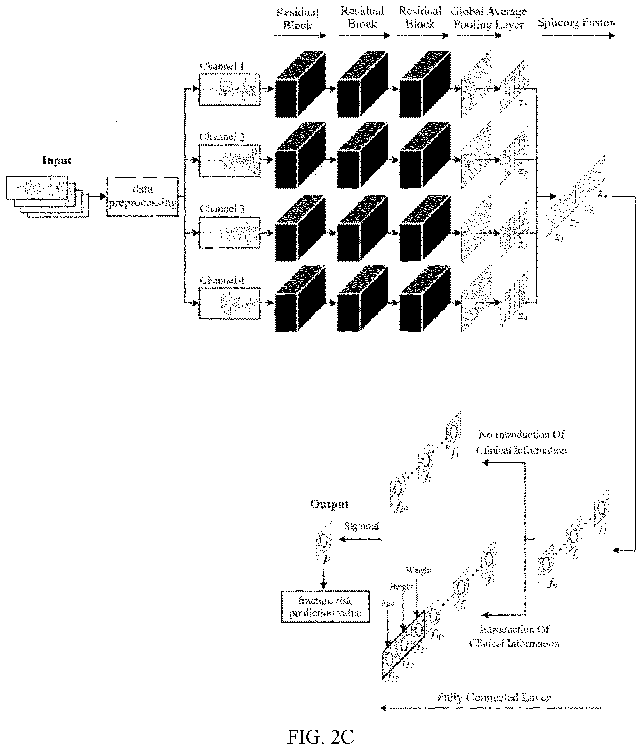

FIG. 2C shows an overall flow chart according to an embodiment of the present disclosure. In FIG. 2C, assuming that the 4-channel bone radio-frequency data of the QUS device is obtained, the 4-channel bone radio-frequency data is first preprocessed. The preprocessed 4-channel bone radio-frequency data is input into the residual module of the corresponding channel of the 4-channel residual sub-network, and the 4-channel bone features corresponding to the 4-channel bone radio-frequency data are extracted. The 4-channel bone features are input into the 4-channel global average pooling layer to perform dimensionality reduction processing. The 4-channel bone features processed by the 4-channel global average pooling layer are input into the cascade layer, so that the 4-channel bone features are axially cascaded along the channel axis to obtain cascade bone features. Training is performed by taking the cascade bone features as the input training data for the decision neural sub-network comprising two fully connected layers and an activation function layer and taking the fracture evaluation value corresponding to the 4-channel bone radio-frequency data as the output training data for the decision neural sub-network, so as to obtain a multi-channel residual neural network model.

The model trained by the above model training method proposed in the present disclosure can obtain an effective bone data decision value. Compared with the prediction model constructed by calculating the quantitative parameters such as SOS value and BUA value of the radio-frequency signal, the model of the present disclosure is more accurate. In addition, the technical solution in the present disclosure only needs to analyze a total of about 60 seconds of radio-frequency data collected at one site to obtain a better model performance, thus minimizing the influence of human factors such as the operators, the collection sites, the bones and the relative position of the ultrasound probe. The technical solution in the present disclosure can also be applied to portable wearable devices, which can be easily promoted to the masses, and provide a more convenient and effective evaluation method for the prevention of osteoporotic fractures in people with reduced bone strength and osteoporosis., to help them get more timely targeted prevention and treatment.

The following are device embodiments of the present disclosure, which may be used to implement the method embodiments of the present disclosure.

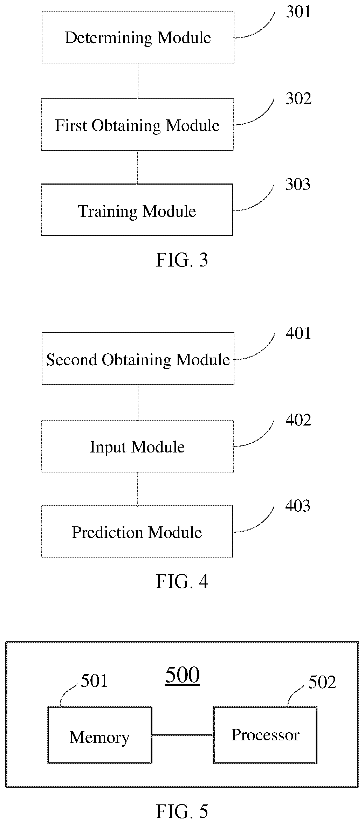

FIG. 3 shows a structural block diagram of a model training device according to an embodiment of the present disclosure. The device can be implemented as part or all of an electronic apparatus through software, hardware, or a combination of both. As shown in FIG. 3, the model training device comprises:

a determination module 301 configured to determine an initial multi-channel residual neural ne
a first obtaining module 302 configured to obtain a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data;

The training module 303 is configured to train the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

As mentioned above, osteoporosis is a metabolic bone disease syndrome characterized by decreased bone mass and destruction of bone microstructure, which can lead to increased bone fragility and susceptibility to osteoporotic fractures. Quantitative ultrasound (QUS) is a bone mineral density measurement technique. Its working principle is to detect bone quality by using the different propagation speeds and attenuations of ultrasound in different bone components. As a non-ionizing technology, QUS has the advantages of low cost, portability, rapidity, and no ionizing radiation, and therefore has good promotional properties. Based on the ultrasonic radio-frequency (RF) signal transmitted to and received from the bone by QUS equipment, parameters such as sound velocity value, broadband ultrasonic attenuation value, rigidity index, quantitative ultrasonic index, etc. can be calculated and output. The above parameters are only some of the characteristic values in the ultrasound radio-frequency signal. If the analysis is only based on the above parameters, a large amount of other information related to bone quality in the ultrasound radio-frequency signal will be lost. However, the ultrasound radio-frequency signal is relatively complex, and it is currently difficult to clarify the potential key variables in the ultrasound radio-frequency signal that may be related to the risk of osteoporotic fractures. Therefore, a model that can more comprehensively and accurately extract the characteristics of ultrasonic radio-frequency signals is urgently needed.

Taking into account the above defects, in this embodiment, a model training device is proposed, which can comprehensively and accurately extract the characteristics of ultrasonic radio-frequency signals, and train a robust multi-channel residual neural network model. This model can be applied in the field of fracture risk prediction to take timely and effective preventive measures, which can reduce the incidence of osteoporotic fractures and reduce patients' pain.

In an embodiment of the present disclosure, the model training device can be implemented as a computer, a computing device, a terminal device, an electronic device, a server, a service cluster and other model training parties for predicting the fracture risk.

FIG. 4 shows a structural block diagram of a fracture risk prediction device according to an embodiment of the present disclosure. The device can be implemented as part or all of an electronic device through software, hardware, or a combination of both. As shown in FIG. 4, the fracture risk prediction device comprises:

a second obtaining module 401 configured to obtain multi-channel bone radio-frequency data obtained by a QUS device;

an input module 402 configured to input the multi-channel bone radio-frequency data into a pre-trained multi-channel residual neural network model to obtain a fracture risk prediction probability;

a prediction module 403 configured to obtain a fracture risk prediction result based on the fracture risk prediction probability.

The technical terms and technical features involved in the above-mentioned device-related embodiments are the same as or similar to the technical terms and technical features mentioned in the above-mentioned method-related embodiments. Explanation and description of the technical terms and technical features involved in the above-mentioned device-related embodiments may be made reference with the above explanation and description of the above-mentioned method-related embodiments, which will not be described again here.

The present disclosure also discloses an electronic apparatus. FIG. 5 shows a structural block diagram of an electronic apparatus according to an embodiment of the present disclosure. As shown in FIG. 5, the electronic apparatus 500 comprises a memory 501 and a processor 502; wherein

The memory 501 is used to store one or more computer instructions, wherein the one or more computer instructions are executed by the at least one processor 502 to implement the steps of the model training method mentioned above.

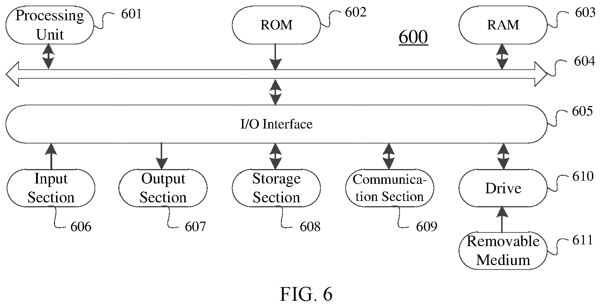

FIG. 6 is a schematic structural diagram of a computer system suitable for implementing a model training method according to an embodiment of the present disclosure.

As shown in FIG. 6, the computer system 600 comprises a processing unit 601, which can perform the processing in the above-mentioned embodiments according to a program which is stored in a read-only memory (ROM) 602 or which is loaded from a storage section 608 into a random-access memory (RAM) 603. Various programs and data required for the operation of the system 600 are also stored in the RAM 603. The processing unit 601, ROM 602 and RAM 603 are connected to each other through a bus 604. An input/output (I/O) interface 605 is also connected to bus 604.

The following components are connected to the I/O interface 605: an input section 606 including a keyboard, a mouse, etc.; an output section 607 including a cathode ray tube (CRT), a liquid crystal display (LCD), etc., and a speaker, etc.; a storage section 608 including a hard disk, etc.; and a communication section 609 including a network interface card such as a LAN card, a modem, or the like. The communication section 609 performs communication processing via a network such as the Internet. A drive 610 is also connected to the I/O interface 605 as needed. A removable medium 611, such as a magnetic disk, an optical disk, a magneto-optical disk, a semiconductor memory, etc., is mounted on the drive 610 as needed, so that a computer program read therefrom is installed into the storage section 608 as needed. Herein the processing unit 601 may be implemented as a processing unit such as a CPU, GPU, TPU, FPGA, or NPU, etc.

In particular, according to embodiments of the present disclosure, the method described above may be implemented as a computer software program. For example, embodiments of the present disclosure comprise a computer program product, which comprises a computer program tangibly embodied on a readable medium thereof, and the computer program comprises program code for executing the method. In such embodiments, the computer program may be downloaded and installed from the network via communication section 609, and/or installed from removable medium 611.

The flowchart and block diagrams in the drawings illustrate the architecture, functionality, and operation of possible implementations of systems, methods and computer program products according to various embodiments of the present disclosure. In this regard, each block in a roadmap or block diagram may represent a module, program segment, or part of code that contains one or more executable instructions. It should also be noted that, in some alternative implementations, the functions noted in the blocks may occur out of the order noted in the drawings. For example, two blocks shown in succession may, in fact, be executed substantially concurrently, or they may sometimes be executed in the reverse order, depending upon the functionality involved. It is also to be noted that each block in the block diagrams and/or flowcharts, and combinations of blocks in the block diagrams and/or flowcharts, can be implemented by a dedicated hardware-based system that performs the specified functions or operations, or can be implemented by using a combination of dedicated hardware and computer instructions.

The units or modules involved in the embodiments described in the present disclosure may be implemented in a software manner, or may be implemented in a hardware manner. The described units or modules may also be provided in the processor, and the names of these units or modules do not constitute a limitation on the units or modules themselves under certain circumstances.

As another aspect, the present disclosure also provides a computer-readable storage medium. The computer-readable storage medium may be the computer-readable storage medium comprised in the device described in the above-mentioned embodiment. It may also be an individual computer-readable storage medium which does not be assembled in a device. The computer-readable storage medium stores one or more programs, and the programs are used by one or more processors to execute the methods described in the present disclosure.

The above description is only a description of the preferred embodiments of the present disclosure and the technical principles applied. Those skilled in the art should understand that the scope of the invention involved in the present disclosure is not limited to technical solutions composed of specific combinations of the above technical features, but should also cover other technical solutions formed by above technical features or any combination of equivalent features without departing from the concept of the invention. For example, a technical solution is formed by replacing the above features with technical features with similar functions disclosed in (but not limited to) this disclosure.

**Claims**

1. A model training method, comprising:

   determining an initial multi-channel residual neural network model;
   obtaining a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data;
   training the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

2. The method according to claim 1, the multi-channel residual neural network model comprises a multi-channel residual sub-network, a multi-channel global average pooling layer, a cascade layer and a decision neural sub-network connected sequentially, wherein:

   the multi-channel residual sub-network is used to extract multi-channel bone features of the multi-channel bone radio-frequency data;
   the multi-channel global average pooling layer is used to utilize global information to perform dimensionality reduction processing on the multi-channel bone features;
   the cascade layer is used to perform cascade processing on the output of the multi-channel global average pooling layer to obtain cascade bone features;
   the decision neural sub-network is used to make decisions based on the cascade bone features.

3. The method according to claim 1, wherein:

   the multi-channel residual sub-network is composed of residual modules of a plurality of channels; the residual module of each channel comprises a plurality of cascaded residual blocks; each residual block comprises a convolution connection branch, a shortcut connection branch, an addition layer, an activation function layer and a max pooling layer; the convolution connection branch comprises a plurality of groups of convolution layers, activation function layers and batch normalization layers; the shortcut connection branch comprises a group of convolution layer, activation function layer and batch normalization layer; the convolution connection branch and the shortcut connection branch are aggregated in the addition layer, and an activation function layer and the max pooling layer are connected behind the addition layer;
   the decision neural sub-network comprises two fully connected layers and an activation function layer, wherein the number of neurons in the first fully connected layer is within the preset number of neurons, and the number of neurons in the second fully connected layer is related to the number of clinical information data categories.

4. The method according to claim 3, said training the initial multi-channel residual neural network model by taking the multi-channel bone radio-frequency data as input and taking the fracture evaluation value label corresponding to the multi-channel bone radio-frequency data as output, comprising:

   inputting the multi-channel bone radio-frequency data into a plurality of cascaded residual blocks of the residual module of the corresponding channel in the multi-channel residual sub-network, wherein the output of the end residual block is the multi-channel bone features corresponding to the multi-channel bone radio-frequency data; inputting the multi-channel bone features into the multi-channel global average pooling layer; inputting the output of the multi-channel global average pooling layer into the cascade layer to obtain the cascade bone features; inputting the cascade bone features into the first fully connected layer and the second fully connected layer in the decision neural sub-network sequentially, and inputting the output of the second fully connected layer into the activation function layer to perform nonlinear calculation, so as to obtain a bone data decision value.

5. The method according to claim 4, said inputting the multi-channel bone radio-frequency data into a plurality of cascaded residual blocks of the residual module of the corresponding channel in the multi-channel residual sub-network comprising:

inputting the multi-channel bone radio-frequency data into the convolution connection branch and the shortcut connection branch of the first residual block of the residual module of the corresponding channel in the multi-channel residual sub-network; inputting the output of the convolution connection branch and the output of the shortcut connection branch into the addition layer, wherein the outputs are added in the addition layer; inputting the output of the addition layer into the activation function layer for processing; inputting the output of the activation function layer into the max pooling layer, and inputting the output of the max pooling layer, which is used as the input of the next residual block, into the convolution connection branch and the shortcut connection branch of the next residual block, until reaching the end residual block.

6. The method according to claim 5, before inputting the multi-channel bone radio-frequency data into a plurality of cascaded residual blocks of the residual module of the corresponding channel in the multi-channel residual sub-network, further comprising:
preprocessing the multi-channel bone radio-frequency data.

7. The method according to any one of claims 1 to 6, the multi-channel residual neural network model performs model training by taking small sample cross entropy as the loss function.

8. The method according to any one of claims 1 to 7, further comprising:

obtaining multi-channel bone radio-frequency data to be decided; inputting the multi-channel bone radio-frequency data to be decided into the multi-channel residual neural network model, to obtain the bone data decision value corresponding to the multi-channel bone radio-frequency data to be decided.

9. The method according to claim 8, further comprising:
performing a preset operation according to the bone data decision value.

10. A model training device, comprising:

a determining module configured to determine an initial multi-channel residual neural network model; a first obtaining module configured to obtain a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data; a training module configured to train the initial multi-channel residual neural network model by taking the multi-channel bone radio-frequency data as input and taking the fracture evaluation value label corresponding to the multi-channel bone radio-frequency data as output, so as to obtain a multi-channel residual neural network model.

11. A fracture risk prediction device, comprising:

a second obtaining module configured to obtain multi-channel bone radio-frequency data obtained by a QUS device; an input module configured to input the multi-channel bone radio-frequency data into a pre-trained multi-channel residual neural network model to obtain a fracture risk prediction probability;
a prediction module configured to obtain a fracture risk prediction result based on the fracture risk prediction probability.

12. An electronic apparatus, which is characteristic in comprising a memory and at least one processor, wherein the memory is used to store one or more computer instructions, wherein the one or more computer instructions are executed by the at least one processor to implement the steps of the model training method according to any one of claims 1 to 9.

13. A computer-readable storage medium which stores computer instructions, , which is characteristic in that the computer instructions are executed by a processor to implement the steps of the model training method according to any one of claims 1 to 9.

14. A computer program product, comprising computer programs/instructions, wherein the computer programs/instructions implement the steps of the model training method according to any one of claims 1 to 9 when the computer programs/instructions are executed by a processor.

determining an initial multi-channel residual neural network model | S101

obtaining a multi-channel residual neural network model training data set, wherein the multi-channel residual neural network model training data set comprises multi-channel bone radio-frequency data obtained by a QUS device, and a fracture evaluation value label corresponding to the multi-channel bone radio-frequency data | S102

training the initial multi-channel residual neural network model by taking the multi-channel bone radio-frequency data as input and taking the fracture evaluation value label corresponding to the multi-channel bone radio-frequency data as output, so as to obtain a multi-channel residual neural network model | S103

FIG. 1

residual block   residual block   residual block

FIG. 2A

**Residual Block**

Input

convolution layer

ReLU activation function layer

batch normalization layer

convolution layer

ReLU activation function layer

batch normalization layer

convolution layer

ReLU activation function layer

batch normalization layer

convolution layer

batch normalization layer

ReLU activation function layer

addition

← ReLU

max pooling layer

Output

FIG. 2B

FIG. 2C

Determining Module — 301

First Obtaining Module — 302

Training Module — 303

FIG. 3

Second Obtaining Module — 401

Input Module — 402

Prediction Module — 403

FIG. 4

500

Memory — 501

Processor — 502

FIG. 5

Processing Unit ~ 601    ROM ~ 602    600    RAM ~ 603

~ 604

I/O Interface ~ 605

Input Section ~ 606    Output Section ~ 607    Storage Section ~ 608    Communica-tion Section ~ 609    Drive ~ 610

Removable Medium ~ 611

FIG. 6

| INTERNATIONAL SEARCH REPORT | International application No. |
| | **PCT/CN2022/080984** |

**A. CLASSIFICATION OF SUBJECT MATTER**

G06T 7/00(2017.01)i

According to International Patent Classification (IPC) or to both national classification and IPC

**B. FIELDS SEARCHED**

Minimum documentation searched (classification system followed by classification symbols)

G06T

Documentation searched other than minimum documentation to the extent that such documents are included in the fields searched

Electronic data base consulted during the international search (name of data base and, where practicable, search terms used)

CNTXT; CNABS; ENTXT; DWPI; WOTXT; USTXT; VEN; CNKI: 残差神经网络, 训练, 多通道, 骨, 标签, 维度, residual neural network, train, multichannel, bone, label, tag, dimension

**C. DOCUMENTS CONSIDERED TO BE RELEVANT**

| Category* | Citation of document, with indication, where appropriate, of the relevant passages | Relevant to claim No. |
|---|---|---|
| X | CN 108399616 A (XI'AN UNIVERSITY OF TECHNOLOGY) 14 August 2018 (2018-08-14) description, paragraphs 54-92 | 1-10, 12-14 |
| Y | CN 108399616 A (XI'AN UNIVERSITY OF TECHNOLOGY) 14 August 2018 (2018-08-14) description, paragraphs 54-92 | 11 |
| Y | KR 101796620 B1 (CHUNG-ANG UNIVERSITY INDUSTRY-ACADEMY COOPERATION FOUNDATION) 13 November 2017 (2017-11-13) description, pages 4-6 | 11 |
| Y | CN 113128580 A (TIANJIN UNIVERSITY) 16 July 2021 (2021-07-16) description, paragraphs 22-51 | 1-14 |
| Y | CN 109376792 A (HEBEI UNIVERSITY OF TECHNOLOGY) 22 February 2019 (2019-02-22) description, paragraphs 3-28 | 1-14 |
| A | CN 111814661 A (XIDIAN UNIVERSITY) 23 October 2020 (2020-10-23) entire document | 1-14 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **29 November 2022** | **14 December 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/080984**

| Patent document cited in search report | | | Publication date (day/month/year) | Patent family member(s) | | | Publication date (day/month/year) |
|---|---|---|---|---|---|---|---|
| CN | 108399616 | A | 14 August 2018 | CN | 108399616 | B | 16 November 2021 |
| KR | 101796620 | B1 | 13 November 2017 | KR | 20160136723 | A | 20 October 2016 |
| CN | 113128580 | A | 16 July 2021 | None | | | |
| CN | 109376792 | A | 22 February 2019 | CN | 109376792 | B | 18 February 2022 |
| CN | 111814661 | A | 23 October 2020 | None | | | |

Form PCT/ISA/210 (patent family annex) (January 2015)